# EUROPÄISCHE PATENTSCHRIFT

(11) **EP 0 553 808 B1**
(45) Veröffentlichungstag und Bekanntmachung des Hinweises auf die Patenterteilung: **07.01.1998**
(21) Anmeldenummer: 93101300.7
(22) Anmeldetag: 28.01.1993
(51) Int. Cl.: B29C 59/04, B29C 55/00, B29K 105/32, B29K 105/28, A61F 13/15

(54) **Luftdurchlässige und flüssigkeitsundurchlässige hintere Schicht für den Gebrauch in körperflüssigkeitsabsorbierenden Artikeln und Verfahren zu ihrer Herstellung**
Breathable and liquidproof bottom layer to be used in body liquid absorbant articles and method of manufacturing same
Couche inférieure, perméable à l'air et imperméable aux liquides, utilisable dans des articles absorbants des liquides corporels et son procédé de fabrication

(30) Priorität: 28.01.1992 JP 38829/92
(43) Veröffentlichungstag der Anmeldung: 04.08.1993
(73) Patentinhaber: UNI-CHARM CORPORATION, Kawanoe-shi Ehime-ken (JP)
(72) Erfinder: Soga, Hiroyuki, Kawanoe-shi, Ehime-ken (JP); Matsushita, Michiyo, Iyomsihima-shime, Ehime-ken (JP)
(74) Vertreter: Sperling, Rüdiger, Dipl.-Ing.

(56) Entgegenhaltungen:
- EP-A- 0 232 060
- EP-A- 0 319 222
- US-A- 5 078 708
- DATABASE WPIL Section Ch, Week 8818, 24. März 1988 Derwent Publications Ltd., London, GB; Class A08, AN 88-122681 & JP-A-63 066 241 ( DAINIPPON PRINTING KK )
- DATABASE WPIL Section Ch, Week 8809, 25. Januar 1988 Derwent Publications Ltd., London, GB; Class A04, AN 88-061346 & JP-A-63 017 941 ( NITTO ELECTRIC IND KK )
- DATABASE WPIL Section Ch, Week 9112, 8. Februar 1991 Derwent Publications Ltd., London, GB; Class A04, AN 91-084439 & JP-A-3 030 934 (UNI CHARM KK )

## Beschreibung

Die Erfindung betrifft eine luftdurchlässige und feuchtigkeitsundurchlässige hintere Schicht zur Verwendung bei Körperflüssigkeit absorbierenden Artikeln. Eine derartige Schicht enthält zumindest eine Polyolefin-Kunststoffschicht. Ferner befaßt sich die Erfindung mit einem Verfahren zur Herstellung einer derartigen Schicht.

Die EP 0 232 060 A2 betrifft einen gasdurchlässigen porösen Film, der ein Polyolefinharz und ein anorganisches Füllmaterial enthält. Durch eine geheizte Prägewalze und eine Materialkontraktion wird der vorher mit einem Oberflächenbehandlungsmittel behandelte und dann wenigstens in einer Richtung gedehnte Film mit einem widerstandsfähigen oder robusten reliefartigen Prägemuster mit einer Mustertiefe von 2 µm bis 3 mm versehen. Das Strecken oder Dehnen, um dadurch Poren zu bilden, kann alternativ auch nach dem Prägen erfolgen.

Die EP 0 319 222 A2 befaßt sich mit Raschelgeräuschen bei Wegwerfprodukten, wie Windeln, Inkontinenzprodukten und Hygienebinden für Frauen, und offenbart eine luftdurchlässige und feuchtigkeitsundurchlässige hintere Schicht dafür aus einem mikroporösen Polymerfilm. Letzterer besteht aus einem kristallisierbaren thermoplastischen Polymer und enthält in seinen Poren einen Raschelgeräusche vermindernden Zusatz. Nach dem Extrudieren eines solchen Films wird dieser durch Dehnen bei einer Temperatur im Bereich von 10 °C bis 10 °C unter der Schmelztemperatur des thermoplastischen Polymers orientiert, um einen lichtundurchlässigen mikroporösen Film zu bilden. Anschließend wird die gesamte Oberfläche des lichtundurchlässigen mikroporösen Films reliefartig geprägt, so daß in bis zu ungefähr 50 % der Gesamtoberfläche des Films durch relativ tiefe Eindrücke in dessen Schicht lichtdurchlässige Bereiche entstehen.

Dieser bekannte Polymerfilm hat somit Poren und lichtundurchlässige und lichtdurchlässige geprägte Bereiche, an denen die Struktur des Polymerfilms stark geschwächt ist, so daR es leicht zu Beschädigungen einer solchen Schicht kommen kann, die dann keinen ausreichenden Auslaufschutz gewährleistet. Die für diesen Fall vorgesehene Verwendung von zwei oder mehr Schichten führt zu einem erhöhten Herstellungsaufwand, einer reduzierten Lichtdurchlässigkeit und einer Behinderung der Luftdurchlässigkeit.

Es ist ferner aus der Praxis bekannt, eine Polyolefin-Kunststoffschicht, wie beispielsweise eine Polyäthylenschicht, als flüssigkeitsundurchlässige Schicht zu verwenden, welche absorbierende Kerne von Körperflüssigkeit absorbierenden Artikeln abdeckt. Die Verwendung einer Polyäthylenschicht, welche durchsichtig oder lichtdurchlässig ausgebildet ist, ermöglichtes, durch Beobachtung der Schicht zu erkennen, ob die absorbierenden Kerne mit Körperflüssigkeit verschmutzt sind. Ferner ist diese Schicht für eine Beurteilung eines Zeitpunktes geeignet, an dem die Körperflüssigkeit absorbierenden Artikel, welche mit Körperflüssigkeiten verschmutzt sind, gegen frische Artikel ausgetauscht werden sollten. Eine derartige Schicht ist jedoch in ihrer Luftdurchlässigkeit relativ unzureichend, und demnach tendieren die Körperflüssigkeit absorbierenden Artikel mit diesen hinteren Schichten dazu, übel zu riechen.

Es ist ferner aus der Praxis bekannt, eine Polyolefin-Kunststoffschicht, wie beispielsweise eine Polyäthylenschicht, mit feinen Partikeln eines anorganischen Füllstoffs, wie beispielsweise Calciumcarbonat, mit einem gewünschten Zugverhältnis zu strecken, um luftdurchlässige feine Poren zu bilden und um eine derartige Schicht als eine luftdurchlässige und flüssigkeitsundurchlässige hintere Schicht zu verwenden, wie es beispielsweise in den Veröffentlichungsanzeigern für japanische Patentanmeldungen Nr. 1985-199037 und 1987-167332 offenbart ist. Diese konventionellen Techniken sind sicherlich geeignet, um zu verhindern, daß die Körperflüssigkeit absorbierenden Artikel während ihres Gebrauchs in Folge verbesserter Luftdurchlässigkeit übel riechen. Während derartige Schichten des Standes der Technik vor der Streckbehandlung eine bestimmte Transparenz zeigen, können die Füllpartikel jedoch, nachdem gestreckt wurde, möglicherweise in die Oberfläche aufsteigen und feine Vorsprünge bilden, welche wiederum die Schichtoberfläche aufrauhen und dadurch ihr optisches Diffusionsvermögen erhöhen. Folglich geht die Transparenz der Schicht verloren, was es für den Benutzer erschwert, zu erkennen, daß ein absorbierender Kern mit Körperflüssigkeiten verschmutzt wurde.

Es ist die Aufgabe der vorliegenden Erfindung, eine luftdurchlässige und feuchtigkeitsundurchlässige hintere Schicht zur Verwendung bei Körperflüssigkeit absorbierenden Artikeln derart zu verbessern, daß sie stabil ist sowie eine geeignete Luftdurchlässigkeit und gleichzeitig eine zur Sichtprüfung eines in dem Artikel enthaltenen Kerns ausreichende Transparenz aufweist.

Diese Aufgabe wird erfindungsgemäß mit einer Schicht nach dem Anspruch 1 gelöst.

Ein erfindungsgemäßes Verfahren zur Herstellung einer derartigen Schicht ist im Anspruch 8 angegeben.

Die Erfindung schafft somit eine hintere Schicht mit gewünschter Transparenz und Luftdurchlässigkeit sowie geeigneter Verwendbarkeit als Bestandteil von Körperflüssigkeit absorbierenden Artikeln, indem eine luftdurchlässige Polyolefin-Kunststoffschicht, welche anorganische Füllpartikel hat und deren Oberfläche zuerst mittels Streckung aufgerauht wurde, einer Thermo-Walzprägebehandlung unterzogen wird, so daß eine teilweise transparente und glatte Oberfläche geschaffen wird.

Verfahrensmäßig sieht die Erfindung demnach zur Herstellung luftdurchlässiger und flüssigkeitsundurchlässiger hinterer Schichten für den Gebrauch bei Körperflüssigkeit absorbierenden Artikeln vor, daß eine anorganische Füllpartikel von 30 - 80 Gew.-% enthaltende Polyolefin-Kunststoffschicht einer Porenbildungsbehandlung, die mindestens eine Streckung umfaßt, unterzogen wird, wobei diese Schicht nicht nur mit feinen luftdurchlässigen Poren versehen, sondern auch zumindest eine Oberfläche der Schicht aufgerauht wird, und wobei anschließend ein Thermo-Walzprägen der Schicht ausgeführt wird, so daß die aufgerauhte Oberfläche im Vergleich zur verbleibenden Oberfläche teilweise geglättet ist.

Ferner schafft die Erfindung vorrichtungsmäßig eine luftdurchlässige aber feuchtigkeitsundurchlässige hintere Schicht für den Gebrauch bei Körperflüssigkeit absorbierenden Artikeln, wobei die hintere Schicht eine Polyolefin-Kunststoffschicht mit anorganischen Füllpartikeln von 30 - 80 Gew-% aufweist, auf mindestens einer Oberfläche der Schicht rauhe Oberflächenzonen mit einer großen Anzahl von feinen, durch Aufrichten der Partikel geformten Vorsprüngen und einer großen Anzahl von feinen luftdurchlässigen Poren aufweist, und die verbleibenden Oberflächenzonen im Vergleich zu den rauhen Oberflächenzonen geglättet sind.

Vorzugsweise bilden die rauhen Oberflächenzonen und die glatten Oberflächenzonen zusammen ein gewünschtes Oberflächenmuster.

Gemäß dieser Erfindung wird die luftdurchlässige und flüssigkeitsundurchlässige hintere Schicht durch Streckung der Polyolefin-Kunststoffschicht, welche anorganische Füllpartikel aufweist, erzielt, so daß die Schichtoberfläche durch die sich zur Oberfläche aufrichtenden Füllpartikel aufgerauht wird, wobei feine luftdurchlässige Poren gebildet werden. Diese oberflächenaufgerauhte Schicht wird thermo-walzgeprägt, wodurch Teile der Schicht mit einer Prägewalze in Berührung kommen und unter einem Druck erweicht oder geschmolzen werden, so daß die rauhe Oberfläche in eine glatte Oberfläche umgewandelt wird.

Die Prägewalze kann mit einem bestimmten Muster versehen sein, um ein entsprechendes Schichtoberflächenmuster zu erhalten, welches aus den glatten Oberflächenzonen und den rauhen Oberflächenzonen zusammengesetzt ist. Die Verwendung dieser, ein derartiges Oberflächenmuster tragenden Schicht als die hintere Schicht, welche zur Abdeckung des absorbierenden Kerns des Körperflüssigkeit absorbierenden Artikels dient, erleichtert die Identifikation des mit Körperflüssigkeit verschmutzten absorbierenden Kerns, da die glatten Oberflächenzonen einerseits eine ausreichende Transparenz haben und es ermöglichen, daß der mit Körperflüssigkeit verunreinigte absorbierende Kern wirksam durch die rauhen Oberflächenzonen abgeschlossen ist, und andererseits durch die optische Diffusion eine unzureichende Transparenz haben und dadurch die besagte Identifikation schwierig ist. Selbst nach einem Thermo-Walzprägen behalten derartige Schichten die feinen luftdurchlässigen Poren zumindest über die rauhen Oberflächenzonen.

Im folgenden werden Ausführungsbeispiele der Erfindung anhand der Zeichnungen näher erläutert, in welchen:
- Fig. 1: eine perspektivische Ansicht einer Wegwerfwindel mit der hinteren Schicht gemäß der Erfindung zeigt;
- Fig. 2: vergrößerte Beispiele (a) und (b) des Oberflächenmusters zeigende Teilansicht zeigen, und
- Fig. 3: ein ein Verfahren zur Herstellung der hinteren Schicht zeigendes schematisches Diagramm zeigt.

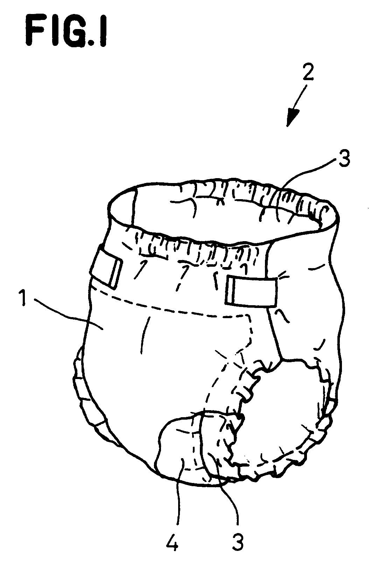

Fig. 1 ist eine teilweise durchbrochen dargesteltte perspektivische Ansicht, welche eine zum bestimmungsgemäßen Gebrauch vorbereitete, aufgerichtete Wegwerfwindel 2 zeigt, wobei die Windel, eine oberflächengemusterte luftdurchlässige hintere Schicht 1 gemäß der Erfindung aufweist. Wie im Stand der Technik umfaßt die Windel 2 eine Oberschicht 3, die hintere Schicht 1 und einen absorbierenden Kern 4, welcher zwischen den Schichten 1, 3 angeordnet ist. Die hintere Schicht 1 definiert die äußere Seite der Windel 2 und hat eine ausreichende Flüssigkeitsundurchlässigkeit, um das Auslaufen von Körperflüssigkeiten zu verhindern.

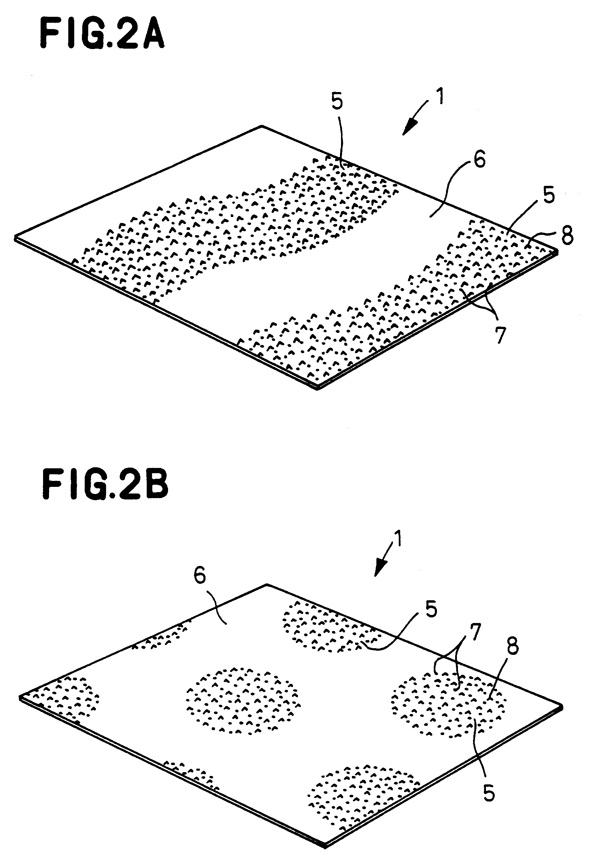

Fig. 2 ist eine perspektivische Ausschnittsansicht, welche in vergrößertem Maßstab die hintere Schicht 1 gemäß der Erfindung zeigt, wobei (a) und (b) zwei Beispiele der hinteren Schicht 1 mit verschiedenen Oberflächenmustern zeigen. Die hintere Schicht 1 ist eine Polyäthylen-Schicht, welche eine Dicke von 50 µm hat und Teile eines anorganischen Füllers, wie beispielsweise Calciumcarbonat oder Bariumsulfat von 30 - 80 Gew-% beinhaltet.

Zumindest eine Oberfläche der hinteren Schicht 1 weist mindestens eine rauhe Oberflächenzone 5 und wenigstens eine relativ dazu glatte Oberflächenzone 6 auf, so daß diese beiden Zonen beispielsweise ein gestreiftes Muster wie es in Figur 2a dargestellt ist oder ein Pünktchenmuster bilden, wie es in Figur 2b dargestellt ist. Die rauhe Oberflächenzone 5 weist eine große Anzahl von feinen Poren 7 mit jeweils einem Durchmesser von 0,03 - 5 µm und eine große Anzahl von feinen Vorsprüngen 8 auf, welche durch Aufrichten der Füllpartikel geformt sind. Das Vorhandensein der feinen Poren 7 macht die rauhe Oberflächenzone luftdurchlässig, erhält aber nahezu die Flüssigkeitsundurchlässigkeit dieser Zone 5, während das Vorhandensein der feinen Vorsprünge 8 eine Abdeckung dieser Zone 5 auf Grund optischer Diffusionen von durchgelassenem Licht und reflektiertem Licht hervorruft. Dagegen weist die glatte Oberflächenzone 6 darauf praktisch keine der feinen Vorsprünge 8 auf, oder wenn sie einige aufweist, sind diese wesentlich niedriger als die feinen Vorsprünge 8 in der rauhen Oberflächenzone 5, so daß die optische Diffusion durchgelassenen Lichtes und reflektierten Lichtes reduziert und dadurch sichergestellt werden kann, daß eine höhere Transparenz erhalten wird, als die der rauhen Oberflächenzone 5. Die glatte Oberflächenzone 6 kann feine Poren 7 haben oder nicht.

Ist die hintere Schicht 1 in einem gebrauchsfertigen Zustand, wie er in Fig. 1 dargestellt ist, kann das Auftreten von Ausscheidungen leicht erkannt und dadurch ein Zeitpunkt zum Wechseln der Windel 2 bestimmt werden, da der mit Körperflüssigkeiten verunreinigte absorbierende Kern durch die glatten Oberflächenzonen 6 sichtbar ist. Dagegen ist es relativ schwer, den verunreinigten absorbierenden Kern durch die rauhen Oberflächenzonen 5 zu sehen, und demnach können diese rauhen Oberflächenzonen 5 genutzt werden, um den verunreinigten absorbierenden Kern 4 zu verbergen. Zu diesem Zweck kann die Anordnung wie auch die Größe der rauhen Oberflächenzonen 5 angemessen ausgewählt werden. Jeweilige Anordungen der rauhen Oberflächenzonen 5 und der glatten Oberflächenzonen 6 können ebenso wie das Verhältnis ihrer Flächen zueinander auch angemessen ausgewählt werden, um ein gewünschtes Oberflächenmuster auf der hinteren Schicht 1 zu erhalten. Es ist Verständlich, daß Fig. 2 sowohl die feinen Poren 7 als auch die feinen Vorsprünge 8 im Verhältnis zu der Dicke der hinteren Schicht 1 in übertriebener Größe darstellt, um ihr Vorhandensein klarzustellen.

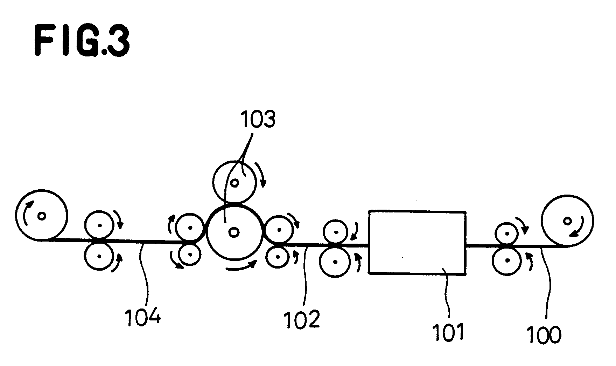

Fig. 3 ist eine schematische Darstellung, welche ein Verfahren zur Herstellung der hinteren Schicht 1 zeigt. Eine Polyäthylenschicht 100 mit einer Dicke von ungefähr 20 µm, welche Partikel eines anorganischen Füllers, beispielsweise Calciumcarbonat oder Bariumsulfat, von 30 - 80 Gew.-% aufweist, wird in eine Streckmaschine 101 eingeführt, durch welche die Schicht 100 sowohl in ihrer Länge als auch in ihrer Breite unter Erwärmung um 100 % gestreckt und dann gekühlt wird, um eine gestreckte Schicht 102 mit feinen luftdurchlässigen Poren 7 zu erhalten. Dabei werden die entgegengesetzt liegenden Oberflächen aufgerauht. Die gestreckte Schicht 102 wird zwischen einem Paar Prägewalzen 103 durchgeführt, welche eine Kombination einer Musterrolle mit einem darauf angeordneten gestreiften Muster, wie es in Figur 2a dargestellt ist, und einer glatten Oberflächenrolle aufweist, wobei die aufgerauhten Oberflächen teilweise durch ihre Aufheizung unter einem bestimmten Druck geglättet werden, um eine luftdurchlässige Schicht 104 zu erhalten, die ein Oberflächenmuster hat, welches durch die rauhen Oberflächenzonen und die glatten Oberflächenzonen bestimmt ist. Anschließend wird diese Schicht 104 geeignet geschnitten, um einzelne hintere Schichten 1 zu erhalten.

Bei einer ausschließlichen Verwendung für Wegwerfwindeln hat die hintere Schicht 1 vorzugsweise eine Dicke von 20 - 80 µm. Der Inhalt der anorganischen Füllpartikel in der hinteren Schicht 1 kann derart angepaßt ausgewählt werden, um eine gewünschte Luftdurchlässigkeit und Transparenz, beispielsweise einen durchsichtigen Charakter, zu erreichen.

Die hintere Schicht 1 gemäß dieser Erfindung ist durch die feinen Poren luftdurchlässig, die über die rauhen Oberflächenzonen verteilt angeordnet sind, welche ferner zur Abdeckung des mit darin abgelagerten und darin absorbierten Körperflüssigkeiten verunreinigten absorbierenden Kerns ausreichend sind und dabei die körperflüssigkeitabsorbierenden Artikel in einer akzeptablen Erscheinung behält. Die glatte Oberflächenzone erlaubt Benutzern den Zeitpunkt eines Austauschs des verschmutzten Körperflüssisgkeit absorbierenden Artikels gegen einen frischen Körperflüssigkeit absorbierenden Artikel zu beurteilen, da die glatten Oberflächenzonen einen durchsichtigen Charakter haben, welcher das Erkennen des verunreinigten absorbierenden Kerns ermöglicht. Die rauhen und glatten Oberflächenzonen können angemessen angeordnet sein, um ein gewünschtes Oberflächenmuster auf der hinteren Schicht zu erzielen. Derartige hintere Schichten können in einfacher Weise durch Streckung von Polyolefin-Kunststoffschichten, welche die anorganischen Füllpartikel beinhalten, und anschließendes Thermo-Walzprägen dieser gestreckten Schichten hergestellt werden.

## Patentansprüche

1. Luftdurchlässige und feuchtigkeitsundurchlässige hintere Schicht zur Verwendung bei Körperflüssigkeit absorbierenden Artikeln, wobei die hintere Schicht zumindest eine Polyolefin-Kunststoffschicht mit 30 bis 80 Gewichtsprozent anorganische Füllpartikel aufweist,
wobei die Polyolefin-Kunststoffschicht (104; 1) erste (5) und zweite Oberflächenzonen (6), von denen die ersten Oberflächenzonen (5) eine geringere Transparenz als die zweiten Oberflächenzonen (6) haben, und eine große Anzahl von luftdurchlässigen Poren (7) aufweist,
wobei die ersten Oberflächenzonen (5) eine rauhe Oberfläche aufweisen, die durch eine große Anzahl von Vorsprüngen (8) gebildet ist, die durch Aufrichten der Partikel geformt sind, und eine große Anzahl von luftdurchlässigen Poren (7) enthalten, und
wobei die zweiten Oberflächenzonen (6) im Vergleich zu den ersten Oberflächenzonen (5) geglättet sind.

2. Schicht nach Anspruch 1, **dadurch gekennzeichnet**, daß die ersten und zweiten Oberflächenzonen (5, bzw. 6) auf der Schicht (104; 1) ein Oberflächenmuster bilden.

3. Schicht nach Anspruch 1 oder 2, **dadurch gekennzeichnet**, daß die Schicht (104; 1) eine Dicke von 20 bis 80 µm hat.

4. Schicht nach einem der vorhergehenden Ansprüche, **dadurch gekennzeichnet**, daß die Dicke der Schicht (104; 1) im Bereich der ersten Oberflächenzonen (5) und der zweiten Oberflächenzonen (6) ohne Berücksichtigung der Vorsprünge (8) auf den ersten Oberflächenzonen (5) zumindest im wesentlichen konstant ist.

5. Schicht nach einem der vorhergehenden Ansprüche, **dadurch gekennzeichnet**, daß die Poren (7) einen Durchmesser von 0,03 bis 5 µm haben.

6. Schicht nach einem der vorhergehenden Ansprüche, **dadurch gekennzeichnet**, daß die anorganischen Füllpartikel Calciumcarbonat oder Bariumsulfat enthalten.

7. Schicht nach einem der vorhergehenden Ansprüche, **dadurch gekennzeichnet**, daß die Vorsprünge (8) durch die durch Streckung der Schicht (100) aufgerichteten anorganischen Füllpartikel gebildet sind.

8. Verfahren zur Herstellung einer Schicht nach einem der vorhergehenden Ansprüche, wobei die in der Schicht (1) enthaltene Polyolefin-Kunststoffschicht (100) mit 30 bis 80 Gewichtsprozent anorganischen Füllpartikeln zur Bildung von feinen luftdurchlässigen Poren (7) und unter Aufrauhung durch Aufrichten der Partikel einer Porenbildungsbehandlung unterzogen wird, die zumindest eine Streckung enthält, und daß anschließend ein Thermo-Walzprägen der Schicht (102) so ausgeführt wird, daß die aufgerauhte Oberfläche in den zweiten Oberflächenzonen (6) im Vergleich zu den ersten Oberflächenzonen (5) geglättet wird.

9. Verfahren nach Anspruch 8, **dadurch gekennzeichnet**, daß die Schicht (100) durch die Streckung in Längs- und Breitenrichtung unter Erwärmung um 100 % gestreckt und dann gekühlt wird.

10. Verfahren nach Anspruch 8 oder 9, **dadurch gekennzeichnet**, daß die gestreckte und gewalzte Schicht (104) in einzelne hintere Schichten (1) geschnitten wird.

## Claims

1. A back sheet, permeable to air and impermeable to liquid, for use in articles absorbing body fluids, wherein the back sheet comprises at least one polyolefin plastics layer with 30 to 80 percent by weight inorganic filler particles, wherein the polyolefin plastics layer (104; 1) has first and second surface zones (5 and 6) and has a large number of pores (7) permeable to air, of which the first surface zones (5) have a smaller transparency than the second surface zones (6), wherein the first surface zones (5) have a rough surface which is formed by a large number of projections (8), which are formed by raising the particles, and a large number of pores (7) permeable to air, and wherein the second surface zones (6) are smooth in comparison with the first surface zones (5).

2. A sheet according to claim 1, characterized in that the first and second surface zones (5 and 6) form a surface pattern on the layer (104; 1).

3. A sheet according to claim 1 or 2, characterized in that the layer (104; 1) has a thickness of 20 to 80 µm.

4. A sheet according to any of the preceding claims, characterized in that the thickness of the layer (104; 1) is at least substantially constant in the region of the first surface zones (5) and the second surface zones (6) without taking into account the projections (8) on the first surface zones (5).

5. A sheet according to any of the preceding claims, characterized in that the pores (7) have a diameter of 0.03 to 5 µm.

6. A sheet according to any of the preceding claims, characterized in that the inorganic filler particles contain calcium carbonate or barium sulphate.

7. A sheet according to any of the preceding claims, characterized in that the projections (8) are formed by the inorganic filler particles raised by stretching the layer (100).

8. A method of making a sheet according to any of the preceding claims, wherein the polyolefin plastics layer (100) included in the sheet (1) with 30 to 80 percent by weight inorganic filler particles is subjected to a pore formation treatment to form fine pores (7) permeable to air and with roughening by raising the particles, which treatment includes at least one stretching, and in that heated roller embossing of the sheet (102) is then so performed that the roughened surface is made smooth in the second surface zones (6) compared with the first surface zones (5).

9. A method according to claim 8, characterized in that the layer (100) is stretched 100% in the length and width directions while heated and is then cooled.

10. A method according to claim 8 or 9, characterized in that the stretched and rolled layer (104) is cut into individual back sheets (1).

## Revendications

1. Couche extérieure perméable à l'air et imperméable aux liquides, pour utilisation dans des articles absorbant les fluides corporels, ladite couche extérieure présentant au moins une couche plastique de polyoléfines renfermant 30 à 80 pour cent en poids de particules de charge minérales, la couche plastique de polyoléfines (104; 1) comprenant des premières (5) et secondes (6) zones superficielles, dont les premières (5) sont moins transparentes que les secondes (6), et présentant un nombre élevé de pores (7) laissant passer l'air, les premières zones superficielles (5) présentant une surface rêche constituée d'un nombre élevé de saillies (8) formées par redressement des particules, et contenant un nombre élevé de pores (7) perméables à l'air, et les secondes zones superficielles (6) étant lissées par opposition aux première zones superficielles (5).

2. Couche suivant la revendication 1, caractérisée en ce que les premières et secondes zones superficielles (5 et 6, respectivement) forment, à la surface de la couche (104; 1), une trame superficielle.

3. Couche suivant la revendication 1 ou 2, caractérisée en ce que la couche (104; 1) présente une épaisseur de 20 à 80 µm.

4. Couche suivant l'une quelconque des revendications précédentes, caractérisée en ce que dans la région des premières zones superficielles (5) et des secondes zones superficielles (6), abstraction faite des saillies (8), l'épaisseur de la couche (104; 1) est au moins sensiblement constante.

5. Couche suivant l'une quelconque des revendications précédentes, caractérisée en ce que les pores (7) présentent un diamètre de 0,03 à 5 µm.

6. Couche suivant l'une quelconque des revendications précédentes, caractérisée en ce que les particules de charge minérales contiennent du carbonate de calcium ou du sulfate de baryum.

7. Couche suivant l'une quelconque des revendications précédentes, caractérisée en ce que les saillies (8) sont formées par les particules de charge minérales redressées par étirage de la couche (100).

8. Procédé de fabrication d'une couche suivant l'une quelconque des revendications précédentes, caractérisé en ce que la couche plastique de polyoléfines (100) contenue dans la couche (1) et renfermant 30 à 80 pour cent en poids de particules de charge minérales, est soumise, en vue de former de fins pores (7) laissant passer l'air et sous création d'une rugosité par étirage des particules, à un traitement générateur de porosité comprenant au moins un étirage, et en ce qu'ensuite, un gaufrage thermique de la couche (102) est exécuté de telle façon que la surface rendue rugueuse des secondes zones superficielles (6) est lissée, par opposition aux premières zones superficielles (5).

9. Procédé suivant la revendication 8, caractérisé en ce que la couche est étirée de 100 % sous chauffage, par l'étirage dans le sens de la longueur et de la largeur, puis refroidie.

10. Procédé suivant la revendication 8 ou 9, caractérisé en ce que la couche étirée et laminée (104) est découpée en couches externes (1) individuelles.
